# EUROPÄISCHE PATENTSCHRIFT

(11) **EP 0 375 863 B1**
(45) Veröffentlichungstag und Bekanntmachung des Hinweises auf die Patenterteilung: **24.11.1993**
(21) Anmeldenummer: 89119276.7
(22) Anmeldetag: 17.10.1989
(51) Int. Cl.: G01N 27/02, G01N 27/06, G01N 27/10, G01N 33/14, G05D 21/02

(54) **Verfahren zum Abfüllen von Bieren**
Beer bottling process
Procédé pour l'emballage de la bière

(30) Priorität: 26.10.1988 DE 3836465
(43) Veröffentlichungstag der Anmeldung: 04.07.1990
(73) Patentinhaber: LANG APPARATEBAU GMBH, 83313 Siegsdorf (DE)
(72) Erfinder: Hantman, Bernhard, D-8221 Bergen (DE)
(74) Vertreter: Wilk, Hans-Christof, Dr.

(56) Entgegenhaltungen:
- DE-C- 1 158 727
- DE-C- 3 400 129
- US-A- 4 733 798
- ADVANCES IN INSTRUMENTATION, Band 41, Teil 1,1986, Seiten 339-352, Research Triangle Park, NC;US; K.M.QUEENEY et al.: "Applications of a microprocessor-based electrodeless conductivity monitor"

## Beschreibung

Die Erfindung betrifft ein Verfahren zum Abfüllen von Bieren und anderen Getränken aus mehreren Tanks durch eine gemeinsame Leitung, in die Frischwasser einspeisbar ist, wobei diese gemeinsame Leitung über Ventile zum Füller, zum Restgetränketank oder zur Abwasserleitung führt.

Aufgrund der erhöhten Anforderungen durch den Gesetzgeber auf nationaler und europäischer Ebene werden die Brauereien immer mehr dazu gezwungen, die Qualität der abgefüllten Produkte noch stärker zu überwachen und zu dokumentieren. Dabei kommt es in erster Linie darauf an, Wasser, Spülwasser, verschiedene Biersorten und verschiedene alkoholfreie Getränke möglichst mit Hilfe technischer Meßeinrichtungen unterscheiden zu können. Bisher war eine zuverlässige Messung bei den hohen Anforderungen an das Meßergebnis aus gerätetechnischer Sicht und aus Hygienegründen nur bedingt möglich. Denn der Meßwertgeber muß voll reinigungs- und sterilisierfähig sowie wartungsfrei und zuverlässig sein. Außerdem muß ein strömungsgünstiger Einbau in Produktionsleitungen möglich sein und die Messung selbst muß auf die Bierüberwachung ausgerichtet sein. Schließlich soll die Überwachung "on-line" im Produktionsprozeß sicher durchgeführt werden.

Diese Aufgaben werden erfindungsgemäß beim eingangs genannten Verfahren dadurch gelöst, daß induktiv der Leitwert der Flüssigkeit und gleichzeitig deren Temperatur in der gemeinsamen Leitung erfaßt wird, daß beim Wechsel der durch die gemeinsame Leitung strömenden Flüssigkeit nur eine derartige Flüssigkeit auf die bisherige folgt, die gegenüber der bisherigen Flüssigkeit einen größeren Leitwertunterschied als die induktiv meßbaren Leitwertschwankungen jedes der beiden Flüssigkeiten aufweist, und daß die Ventile in Abhängigkeit von dem gemessenen Leitwert geöffnet und geschlossen werden.

Die Leitwertmessung hat schon seit einigen Jahren ihren festen Platz an den Flaschenreinigungsmaschinen und den Anlagen zur Konzentrationsüberwachung von Reinigungsmittellösungen und zur Medientrennung von Wasser und Reinigungsmittellösung.

So wurde der Einsatz einer induktiven Leitwertmessung mit Temperaturkompensation zur Kontrolle der NaOH-Konzentration in Tanks der Brauindustrie vorgeschlagen (Advances in Instrumentation, vol.41, Teil 1, 1986, ISA, (Research Triangle Park, NC, US), K.M. Queeney et al.: "applications of a microprocessor-based electrodeless conductivity monitor", Seiten 339-352).

Die DE-C-34 00 129 offenbart ein Verfahren zum Überwachen von Milch, Bier, Fruchtsäften oder dergleichen in Hinblick auf Verunreinigungen durch Säuren, Laugen oder Wasser während der Abfüllung, bei dem der Leitwert induktiv gemessen und eine Alarmeinrichtung betätigt wird, wenn der Leitwert gewisse Schwellwerte über- oder unterschreitet.

Mit dem erfindungsgemäßen Meßgerät kann erreicht werden, daß beim Abfüllen der Getränke keine mit Wasser verdünnten Biere oder Biere mit Resten von Reinigungsmittellösungen zur Abfüllung gelangen, ohne daß zuviel unverdünntes und unverschmutztes Bier in den Abwasserkanal gegeben wird.

Da die Leitwerte der hier in Frage stehenden Flüssigkeiten stark temperaturabhängig sind, wird gleichzeitig die Temperatur der jeweiligen Flüssigkeit erfaßt. Mit Hilfe von vorher ermittelten Temperatur-Koeffizienten der Flüssigkeiten können dann Leitwert-Schwankungen aufgrund von Temperaturschwankungen manuell oder automatisch korrigiert werden.

In dem erfindungsgemäßen Verfahren ist es gelungen, zum einen den Hygieneanforderungen gerecht zu werden und zum anderen die bei Lebensmittelverarbeitung erforderliche erhöhte Genauigkeit, die insbesondere durch die gesetzlichen Grenzwerte vorgegeben ist, zu erreichen und dennoch durch genaueres Umschalten der Ventile Einsparungen wegen eines geringeren Verlustes an Getränken beim Wechsel der Flüssigkeiten und eine Verringerung des mit den Getränken belasteten Abwassers zu erreichen.

Sehr schnelle, wenn auch relativ kleine Temperatursprünge können die Meßwerte beeinflussen, wenn der Temperaturfühler, z.B. ein Platintemperaturfühler, nicht direkt in das Meßmedium ragt und nicht gut umströmt wird. Daher wird vorgeschlagen, daß die Temperatur der durch die gemeinsame Leitung strömenden Flüssigkeit mittels eines Temperaturfühlers mit kurzer Ansprechzeit und kleiner Masse gemessen wird.

Vorteilhaft ist außerdem, wenn ein zur Temperaturmessung frei in die Flüssigkeit ragender Temperaturfühler in unmittelbarer Nähe des Leitwertmeßfühlers verwendet wird.

Bei längerandauernden Unterbrechungen des Flüssigkeitsstroms, z.B. durch ein Schließen des Ventils beim Abfüllen, kann es im Meßkanal der Leitwert-Sonde zu einer geringfügigen Entwicklung von Kohlendioxid-Bläschen kommen, die den Leitwert reduziert. Damit bei geschlossenem Ventil der Leitwert nicht von der angeschlossenen Steuereinheit erfaßt wird, wird vorgeschlagen, daß ein Ventil zum Unterbrechen des Flüssigkeitsstroms vorgesehen ist und daß der Leitwert nur bei Strömung der Flüssigkeit in der gemeinsamen Leitung gemessen wird.

Es hat sich herausgestellt, daß die Schwankungsbreiten der Leitwerte bei den unterschiedlichen Biersorten sowie Sorten von alkoholfreien Getränken zum Teil überlappen und zum anderen Teil deutlich voneinander abgesetzt sind. Daher ist eine genaue Unterscheidung sämtlicher Sorten untereinander, also jeder Sorte von jeder Sorte, nicht möglich. Dennoch können die Sorten, die sich in ihrer Schwankungsbreite nicht überlappen, signifikant unterschieden werden. So läßt sich eine Bier-Biertrennung dann problemlos durchführen, wenn durch eine gezielte Abfüllfolge Biere mit deutlicher Leitwertabweichung hintereinander gefahren werden.

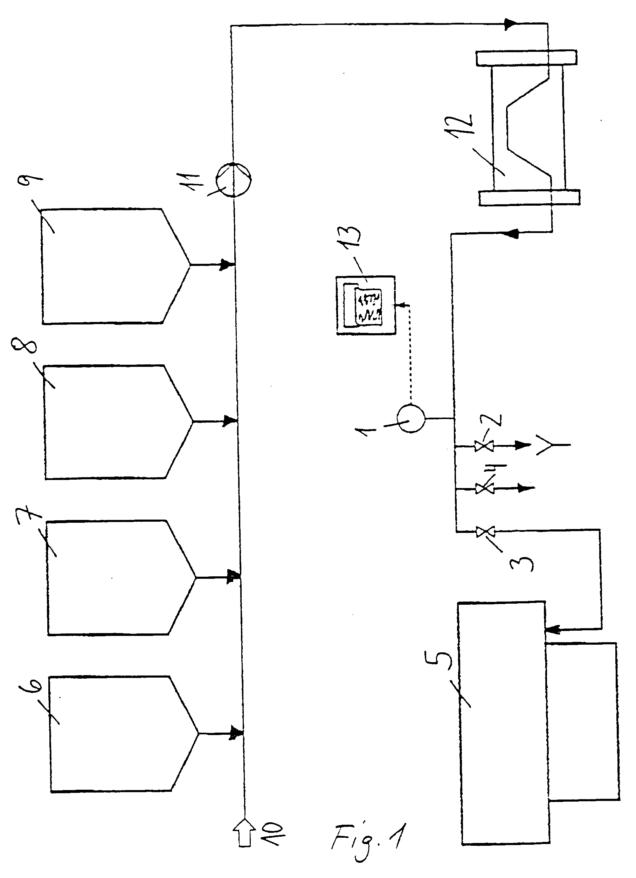

Um die gemessenen Leitwerte in eine Steuerung des Produktionsprozesses umzusetzen, sind mehrere Möglichkeiten gegeben. Die kostengünstigste Variante ist der Einbau eines Leitwertmeßgerätes mit Anzeige direkt an der Meßstelle am Füller, wobei die Ventilumstellung nach Leitwertanzeige und optischer Kontrolle vorgenommen wird. Allgemein wird daher bei dem erfindungsgemäßen Verfahren vorgeschlagen, wobei die die Flüssigkeitsströme regelnde Ventile manuell betätigbar sind, daß zum Messen des Leitwerts ein Meßgerät mit optischer Anzeige des Leitwerts eingesetzt wird. Damit läßt sich sowohl eine Wasser-Biertrennung, wie auch die Bier-Bier-Unterscheidung vornehmen. Ein Ausführungsbeispiel zu diesem Vorschlag ist in Figur 1 dargestellt.

Andererseits ist es jedoch auch möglich, eine Automatisierung der Phasentrennung zu realisieren. Dazu werden Grenzwertgeber zur Ventilumschaltung eingesetzt. Beim erfindungsgemäßen Verfahren, das auf das Abfüllen von Bieren und anderen Getränken angewandt wird, wobei die die Flüssigkeitsströme regelnden Ventile ansteuerbar sind, die die Flüssigkeiten in den Abwasserkanal oder den Flaschen- oder Faß-Füller leiten, wird daher vorgeschlagen, daß die zum Ableiten der Flüssigkeit in den Füller, den Restgetränketank oder die Abwasserleitung dienenden Ventile beim Erreichen eines ersten Leitwerts vom Abwasserkanal auf den Füller und umgekehrt umgeschaltet werden und daß diese Ventile beim Erreichen eines zweiten Leitwerts vom Abwasserkanal auf den Restgetränketank und umgekehrt umgeschaltet werden. Dieser Vorschlag ist besonders wichtig für das Abfüllen der alkoholfreien Getränke, deren Leitwert im Vergleich zu den Bieren nur relativ wenig über dem Leitwert des Betriebswassers liegt. Das Schaltniveau ist hier zwischen dem Leitwert des Betriebswassers und den niedrigsten Leitwerten der alkoholfreien Getränke gelegt.

Durch das Umschalten beim Erreichen des zweiten Leitwerts werden die Verluste durch Ableiten der Flüssigkeit in den Restbiertank verringert.

Beim Ansteigen des Leitwertes auf das zweite Schaltniveau erfolgt eine Signalgabe zur Umschaltung von dem Kanal auf den Restbiertank und beim Ansteigen des Leitwertes auf das erste Schaltniveau erfolgt eine Umschaltung auf den Füller. Umgekehrt verhält es sich bei einem abfallenden Leitwert. Das erste Schaltniveau liegt dabei unterhalb der Schwankungsbreite der Einzelgetränke und höher als das zweite Schaltniveau.

Um die Fehlersicherheit bei den verschiedenen Getränkesorten zu erhöhen, wird beim erfindungsgemäßen Verfahren vorgeschlagen, das daß bei Flüssigkeiten mit ähnlichen Leitwerten beim Erreichen des gleichen ersten und/oder zweiten Leitwerts umgeschaltet wird. Es werden also Flüssigkeiten mit ähnlichen Leitwerten in je einer Gruppe zusammengefaßt, wobei für jede Gruppe das gleiche erste und/oder zweite Schaltniveau vorgesehen ist. Manuell läßt sich die Gruppe, zu der die abzufüllende Getränkesorte gehört, dann an einem Schaltschrank vorwählen, um bestimmte Schaltniveaus zur Ventilumschaltung einzustellen.

In einer weiteren Ausgestaltung der Erfindung ist eine zentrale Überwachung für mehrere Füller möglich und vorteilhaft. Dabei wird vorgeschlagen, daß das Meßsignal über eine freiprogrammierbare Steuerung mit Analogwertverarbeitung ausgewertet wird. Durch die entsprechende Programmierung läßt sich eine weitere Differenzierung der Schaltwertniveaus wirtschaftlich vornehmen. Zudem bietet sich hier in Verbindung mit einem Durchflußzähler eine Zulaufmengenerfassung mit Zuordnung (Kanal/Restbier/Füller) und Registrierung an.

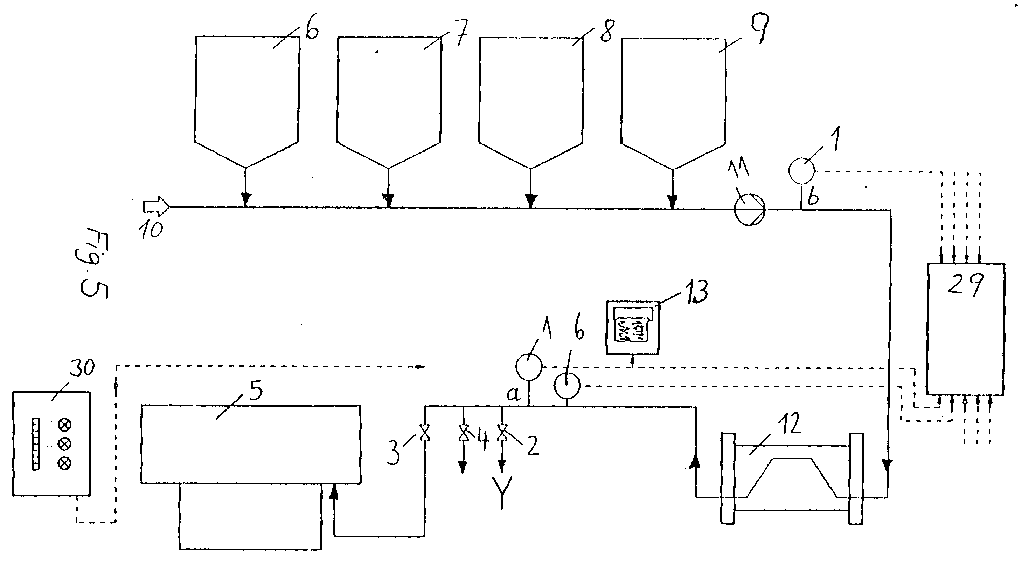

Das erfindungsgemäße Meßverfahren wird besonders genau, wenn die Ventile über die Differenzen der Meßwerte mehrerer Meßstellen angesteuert werden. Diese aufwendigere Meßwerterfassungs- und Auswertesystem basiert auf einem Meßwertvergleich von z.B. zwei Meßstellen, z.B. in einem Abfüllsystem, wobei die Steuerung der Ventilschaltungen nicht durch Absolutwerte vorgegeben werden, sondern sich nach den prozentualen Abweichungen der Meßwerte richten. Das sind die Meßstellen a und b in Figur 5.

Daher wird vorgeschlagen, daß der Leitwert an mehreren Meßstellen der gemeinsamen Leitung gleichzeitig gemessen wird und daß die Ventile in Abhängigkeit von den Leitwertdifferenzen der Meßstellen umgeschaltet werden. Dieses Verfahren gewährleistet eine wesentlich höhere Genauigkeit beim Umschalten, da durch die Differenzmessung die Schwankungsbreiten innerhalb einer Biersorte nicht relevant sind.

Dadurch lassen sich auch Biersorten ohne Zwischenspülung sehr exakt voneinander trennen. Ebenso wird eine genauere Erfassung von Wasserverdünnungen und ein sicheres Erkennen auch geringer Mengen von Reinigungs- und Desinfektionsmitteleinbrüchen erreicht.

Ein Ausführungsbeispiel der Erfindung wird anhand der Zeichnungen nachfolgend näher beschrieben. Es zeigen
- Figur 1:: Ein schematisches Fließbild einer Abfüllanlage, wobei das erfindungsgemäße Verfahren angewendet wird,
- Figur 2:: ein Diagramm für die Leitwerte von Betriebswasser und alkoholfreien Getränken,
- Figur 3:: ein Balkendiagramm mit den Leitwerten von mehreren Biersorten,
- Figur 4:: die Stammwürze-Gehalte mehrerer Biersorten und
- Figur 5:: ein schematisches Fließbild einer Abfüllanlage, wobei die Ventile durch die Differenzen der Leitwerte mehrerer Meßstellen gesteuert werden.

In Figur 1 sind mehrere Drucktanks (6,7,8,9) dargestellt, die in eine gemeinsame Leitung münden. In diese Leitung kann auch Frischwasser (10) eingespeist werden. Die in der Leitung befindliche Flüssigkeit wird über eine Pumpe (11) durch einen Kurzzeiterhitzer (12) und über ein Ventil (3) zum Flaschenfüller (5) gepumpt. Statt zum Flaschenfüller (5) kann die Flüssigkeit auch über ein Ventil (2) bzw. ein Ventil (4) in den Abwasserkanal bzw. in einen Restbiertank gefördert werden.

Kurz bevor die Flüssigkeit die Ventile (2), (4) und (3) erreicht, passiert sie die Meßstelle des Meßgerätes (1), das an einen Schreiber (13) angeschlossen ist. Auf dem Linienschreiber (13) werden der Leitwert und die Temperatur als Analogsignale zwischen 0 und 20 Milliampere aufgezeichnet und ausgewertet.

Erfindungsgemäß sind folgende Unterschiede reproduzierbar zu erfassen:
Der Unterschied zwischen Wasser, Bier und alkoholfreien Getränken; der Unterschied zwischen einer Biersorte a und einer Biersorte b; Verdünnungen im Bier durch Wasser und Vermischungen im Bier durch Reinigungslösungen.

Aus wirtschaftlicher Sicht der Brauerei sind die ersten zwei Punkte wichtig, um bei Ausschubvorgängen die Spülverluste zu reduzieren und die CSB-Belastung durch Getränkereste zu minimieren.

Die letzten beiden Aspekte sind vor allem unter der verschärften Produzentenhaftung zu sehen, um festzustellen, ob die Getränke eventuell mit Reinigungslösungen oder Wasser vermengt sind.

In der Ausführung der Erfindung wurden bei sämtlichen Bieren und alkoholfreien Getränken im Labor mit einem geeigneten Meßgerät die Temperatur-Koeffizienten zwischen Abfülltemperatur und Bezugstemperatur ermittelt. Die gemessenen TK-Werte dieser Getränke schwanken dabei je nach Sorte um etwa 0,07 % pro Grad Celsius um einen Mittelwert von 2,32 %/Grad Celsius, welcher für die Leitwertmessungen digitalgenau eingestellt wurde.

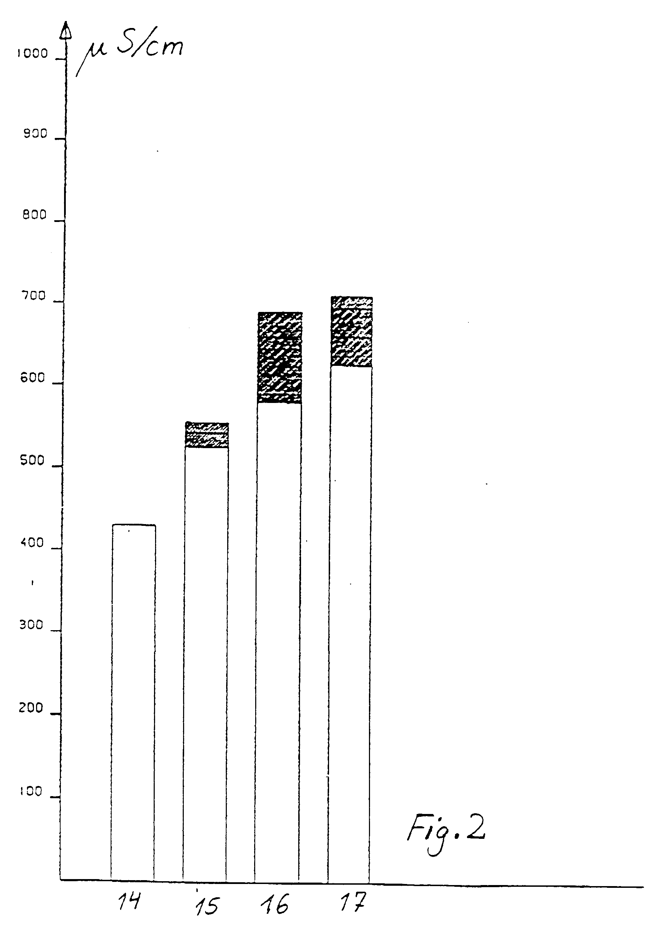

In dem Balkendiagramm von Figur 2 sind die Leitwerte von Betriebswasser (14), Zitronenlimonade (15), Orangenlimonade (16) und Spezi (17) dargestellt. Die Leitwerte dieser Getränke liegen deutlich über dem des Betriebswassers, so daß hier ohne weiteres eine Phasentrennung zwischen Produkt und Spülwasser möglich ist. Die Leitwerte sind in Figur 2 in der Einheit Mikrosiemens pro Zentimeter dargestellt.

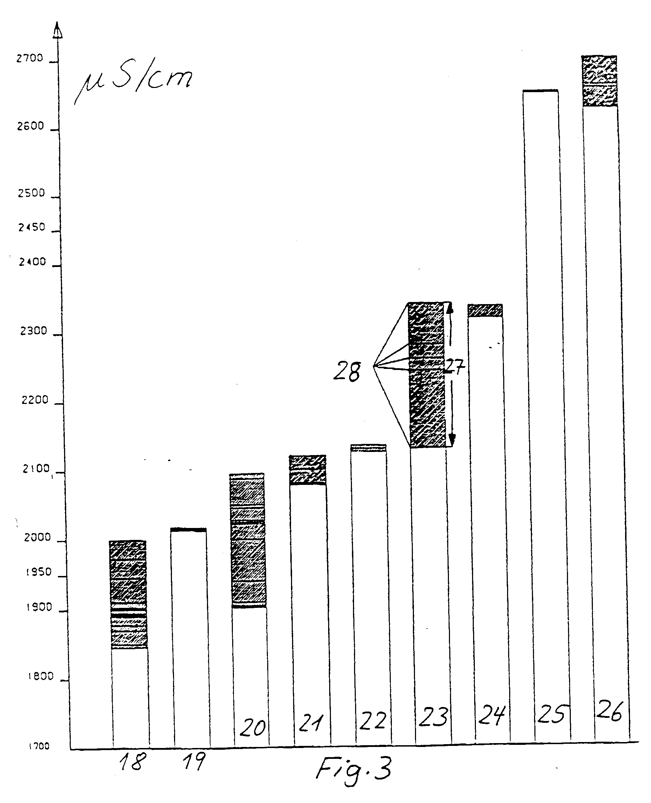

In Figur 3 sind im Balkendiagramm die Leitwerte der gemessenen Biersorten Hefeweißbier (18), Hell Lager (19), Pils (20), Hell Export (21), Dunkel (22), Märzen (23), Heller Bock (24), Doppelbock (25) und alkoholfreies Weißbier (26) dargestellt. Das schraffierte Feld zeigt die Schwankungsbreite je Sorte bei unterschiedlichen Abfülltagen, wobei ein waagerechter Querstrich ein Einzelergebnis darstellt. Die Werte pro Abfüllcharge sind jedoch etwa konstant. Die Schwankungsbreite ist mit (27), und die einzelnen Meßwerte beim Märzenbier (23) mit (28) bezeichnet.

Je nach Sorte liegen die Biere mit Leitwerten zwischen 1800 und 2700 Mikrosiemens/Zentimeter deutlich über dem Betriebswasser mit 430 Mikrosiemens/Zentimeter Bei dieser Differenz läßt sich jede Sorte zuverlässig von Wasser und der Mischphase Wasser-Bier unterscheiden. Allerdings schwanken die Absolutwerte der Biere innerhalb einer Sorte an verschiedenen Abfülltagen und aus unterschiedlichen Drucktanks doch erheblich, zum Teil bis zu 10 %. Die Ursache dafür dürfte bei den Brauprozeßparametern, vor allem im Sudhausbereich, liegen.

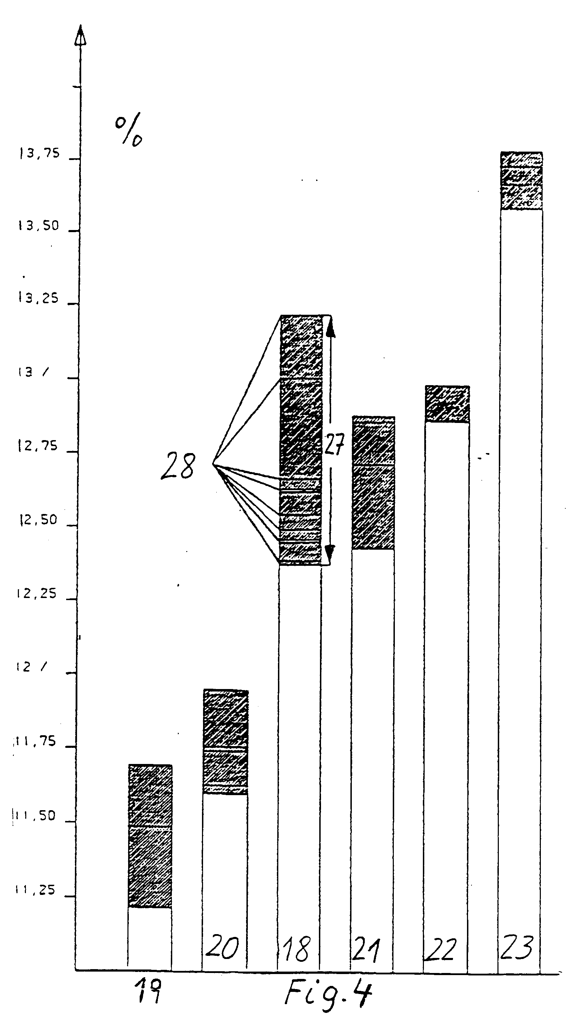

In Figur 4 ist der Stammwürzegehalt einiger Biersorten in Prozent dargestellt. Die verwendeten Bezugszeichen entsprechen denen in Figur 3. Wie zu erkennen ist, lassen die Vergleichsanalysen von den Stammwürze-Gehalten mit den Leitwerten der Biersorten keinen Zusammenhang zwischen dem Stammwürzegehalt und dem Leitwert erkennen. Die Leitwertmessung läßt sich damit nicht auf eine Stammwürzegehaltsmessung zurückführen.

Wie schon oben vorgeschlagen, ist es vorteilhaft, die verschiedenen abzufüllenden Getränke in Gruppen mit ähnlichen Leitwerten einzuteilen. Z.B. kann bei den in Figur 2 und 3 gezeigten Leitwerten die Gruppeneinteilung wie folgt aussehen:

**Tabelle 1**

| | | |
|---|---|---|
| Gruppe 1 | alle alkoholfreien Getränke | Schaltniveau 1 bei 500 µS/cm |
| Gruppe 2 | Hefeweißbier | Schaltniveau 2 bei 600 µS/cm |
| | Hell Lager | |
| | Pils | Schaltniveau 1 bei 1800 µS/cm |
| Gruppe 3 | Hell Export | Schaltniveau 2 bei 600 µS/cm |
| | Dunkel | |
| | Märzen | |
| | Heller Bock | Schaltniveau 1 bei 2000 µS/cm |
| Gruppe 4 | Doppelbock | Schaltniveau 2 bei 600 µS/cm |
| | Weißbier alkoholfrei | Schaltniveau 1 bei 2500 µS/cm |

Im Vergleich zu anderen Meßverfahren, die direkt oder indirekt auf dem Stammwürzegehalt oder dem Trübungsgrad basieren, bietet die induktive Leitwertmessung eine mindestens ebenso genaue, aber wesentlich kostengünstigere Alternative. Brauprozeßbedingt schwanken die Stammwürze-Gehalte und überschneiden sich auch innerhalb der Biersorten, wie Figur 4 zeigt.

Aufgrund der Möglichkeiten der variablen Meßwertauswertung durch freiprogrammierbare Steuerungen kann das Leitwertmeßsystem je nach Anforderung ausgebaut werden. Zudem wird eine Warnung bei Reinigungs- oder Desinfektionsmittelkontamination ermöglicht.

Wie an der bereits oben beschriebenen Einsatzstelle "Flaschenabfüller" ist eine Messung und Trennung in den Produktionsbereichen
Würzekühlung (Trennung von Wasser, Würze, Wasser)
Filtration (Trennung Wasser, Bier, Bier, Wasser)
Flaschenfüller (Trennung Wasser, Bier, Bier, Wasser, alkoholfreie Getränke, Wasser) und
Faß-/Keg-Füllung (Trennung von Wasser, Bier, Bier, Wasser)
nach den gleichen Verfahrensgesichtspunkten sinnvoll und wirtschaftlich.

In Figur 5 ist eine Überwachung des Abfüllvorgangs mit Hilfe der Differenzwertverarbeitung dargestellt. Die Bezugszeichen haben die gleiche Bedeutung wie in Figur 1. Zusätzlich ist in Figur 5 ein Zentralsteuerschrank (29), an den die Leitwertmeßgeräte (1) und der Temperaturfühler (6) angeschlossen sind, sowie ein Bediengehäuse (30) mit einem Anschluß an die Zentralsteuerung (29) dargestellt. Hier wird der Leitwert an mehreren unterschiedlichen Punkten a, b der Leitung gemessen und die Differenz der Meßwerte gebildet. Vorher wird der am Ventil abgenommene Meßwert um den Temperaturfaktor korrigiert. Dazu wird in der Nähe dieser Meßstelle b die Temperatur der Flüssigkeit durch den Thermofühler (6) gemessen.

Durch den Einbau des Leitfähigkeitsmeßgerätes am Abfülleinlauf ist eine Unterscheidung von Bier und alkoholfreien Getränken zu Wasser durch die Leitfähigkeitsmessung möglich. Wichtig für den Brauereieinsatz ist die erfindungsgemäße Erfüllung folgender Kriterien:
Das Leitfähigkeitsmeßsystem erfüllt die Anforderung der Bierüberwachung, ist wartungsfrei und zuverlässig, voll reinigungs- und sterilisierfähig und der Einbau in Produktleitungen ist zulässig. Eine Unterscheidung von Biersorten kann dann vorgenommen werden, wenn der Produktionsablauf der Brauerei ein Ausschieben von Bier mit Bier unterschiedlicher Leitwertniveaus zuläßt.

## Patentansprüche

1. Verfahren zum Abfüllen von Bieren und anderen Getränken, bei dem die Getränke aus mehreren Tanks (6,7,8,9) durch eine gemeinsame Leitung, in die Frischwasser (10) einspeisbar ist und die über Ventile (2,3,4) zum Füller (5), zum Restgetränketank und zur Abwasserleitung führt, abgefüllt werden, wobei
induktiv der Leitwert der Flüssigkeit und gleichzeitig deren Temperatur in der gemeinsamen Leitung erfaßt wird, wobei beim Wechsel der durch die gemeinsame Leitung strömenden Flüssigkeit nur eine derartige Flüssigkeit auf die bisherige folgt, die gegenüber der bisherigen Flüssigkeit einen größeren Leitwertunterschied als die induktiv meßbaren Leitwertschwankungen jedes der beiden Flüssigkeiten aufweist, und wobei die Ventile (2,3,4) in Abhängigkeit von dem gemessenen Leitwert geöffnet und geschlossen werden.

2. Verfahren nach Anspruch 1,
dadurch gekennzeichnet,
daß die Temperatur der durch die gemeinsame Leitung strömenden Flüssigkeit mittels eines Temperaturfühlers (6) mit kurzer Ansprechzeit und kleiner Masse gemessen wird.

3. Verfahren nach Anspruch 1,
dadurch gekennzeichnet,
daß ein zur Temperaturmessung frei in die Flüssigkeit ragender Temperaturfühler in unmittelbarer Nähe des Leitwertmeßfühlers verwendet wird.

4. Verfahren nach Anspruch 1, wobei ein Ventil zum Unterbrechen des Flüssigkeitsstroms vorgesehen ist,
dadurch gekennzeichnet,
daß der Leitwert nur bei Strömung der Flüssigkeit in der gemeinsamen Leitung gemessen wird.

5. Verfahren nach Anspruch 1, wobei die die Flüssigkeitsströme regelnde Ventile manuell betätigbar sind,
dadurch gekennzeichnet,
daß zum Messen des Leitwerts ein Meßgerät (1) mit optischer Anzeige des Leitwerts eingesetzt wird.

6. Verfahren nach Anspruch 1,
dadurch gekennzeichnet,
daß die zum Ableiten der Flüssigkeit in den Füller (5), den Restgetränketank oder die Abwasserleitung dienenden Ventile (2,3) beim Erreichen eines ersten Leitwerts vom Abwasserkanal auf den Füller (5) und umgekehrt umgeschaltet werden und daß diese Ventile (2,4) beim Erreichen eines zweiten Leitwerts, der unterhalb des ersten Leitwerts liegt, vom Abwasserkanal auf den Restgetränketank und umgekehrt umgeschaltet werden.

7. Verfahren nach Anspruch 6,
dadurch gekennzeichnet,
daß bei Flüssigkeiten mit ähnlichen Leitwerten beim Erreichen des gleichen ersten und/oder zweiten Leitwerts umgeschaltet wird.

8. Verfahren nach Anspruch 1,
dadurch gekennzeichnet,
daß das Meßsignal über eine freiprogrammierbare Steuerung mit Analogwertverarbeitung ausgewertet wird.

9. Verfahren nach Anspruch 1,
dadurch gekennzeichnet,
daß der Leitwert an mehreren Meßstellen der gemeinsamen Leitung gleichzeitig gemessen wird und daß die Ventile (2,3,4) in Abhängigkeit von den Leitwertdifferenzen der Meßstellen umgeschaltet werden.

## Claims

1. Method for the bottling of beers and other beverages, in which the beverages are bottled out of several tanks (6, 7, 8, 9) through a common duct, into which fresh water (10) can be fed and which leads by way of valves (2, 3, 4) to the filler (5), to the residual beverage tank and to the waste water duct, wherein the conductance of the liquid is detected inductively and at the same time its temperature in the common duct, wherein on a change of the liquid flowing through the common duct, only such a liquid follows on the previous one as displays a greater conductance difference from the previous liquid than the inductively measurable conductance fluctuations of each of both the liquids and wherein the valves (2, 3, 4) are opened and closed in dependence on the measured conductance.

2. Method according to claim 1, characterised thereby, that the temperature of the liquid flowing through the common duct is measured by means of a temperature sensor (6) of short response time and small mass.

3. Method according to claim 1, characterised thereby, that a temperature sensor projecting freely into the liquid in the immediate proximity of the conductance measuring sensor is used for the temperature measurement.

4. Method according to claim 1, wherein a valve is provided for interrupting the flow of liquid, characterised thereby, that the conductance is measured only during flow of liquid in the common duct.

5. Method according to claim 1, wherein the valves regulating the flows of liquid are actuable manually, characterised thereby, that a measuring instrument (1) with optical indication of the conductance is used for the measurement of the conductance.

6. Method according to claim 1, characterised thereby, that the valves (2, 3), which serve for conducting the liquid away into the the filler (5), to the residual beverage tank and to the waste water duct, are switched over from the waste water duct to the filler (5) and conversely on reaching a first conductance and that these valves (2, 4) are switched over from the waste water duct to the residual beverage tank and conversely on reaching a second conductance which lies below the first conductance.

7. Method according to claim 6, characterised thereby, that in the case of liquids with similar conductances, switching over is done on reaching the same first and/or second conductance.

8. Method according to claim 1, characterised thereby, that the measurement signal is evaluated by way of a freely programmable control with analog value processing.

9. Method according to claim 1, characterised thereby, that the conductance is measured at several measurement points of the common duct at the same time and that the valves (2, 3, 4) are switched over in dependence on the conductance differences of the measurement points.

## Revendications

1. Procédé pour la mise en bouteilles de bières et autres boissons, dans lequel les boissons sont tirées de plusieurs réservoirs (6, 7, 8, 9) et circulent dans une conduite collective qui peut être alimentée en eau fraîche (10) et qui conduit à l'installation de remplissage (5), au réservoir recevant la boisson restante et à la conduite d'eau résiduaire par l'intermédiaire de vannes (2, 3, 4), procédé par lequel la conductance du liquide et en même temps, sa température dans la conduite collective, sont prises par induction, et, dans lequel lorsqu'il y a un changement de liquide circulant dans la conduite collective, le liquide qui succède à celui qui coulait jusqu'alors, doit présenter, par rapport à ce liquide précédent, un écart de conductance supérieur aux fluctuations de conductance, mesurables par induction, de chacun des deux liquides, les vannes (2, 3, 4) étant ouvertes et fermées en fonction de la conductance mesurée.

2. Procédé selon la revendication 1, caractérisé par le fait que la température du liquide circulant dans la conduite collective est mesurée au moyen d'un sondeur de température (6) ayant un temps de réaction court et une masse faible.

3. Procédé selon la revendication 1, caractérisé par le fait qu'un sondeur de température libre se dressant dans le liquide pour mesurer la température est utilisé à proximité immédiate du sondeur de mesure de la conductance.

4. Procédé selon la revendication 1, pour lequel une vanne est prévue pour interrompre l'écoulement de liquide, caractérisé par le fait que la conductance n'est mesurée que lors de l'écoulement du liquide dans la conduite collective.

5. Procédé selon la revendication 1, pour lequel les vannes régulatrices des écoulements de liquide peuvent être commandées manuellement, caractérisé par le fait que, pour mesurer la conductance, on utilise un appareil de mesure (1) avec indications optiques de la conductance.

6. Procédé selon la revendication 1, caractérisé par le fait que, au moment où une première valeur de la conductance est atteinte, les vannes (2, 3) servant à dériver le liquide dans l'installation de remplissage (5), dans le réservoir recevant les boissons restantes ou dans la conduite d'eau résiduaire, sont commutées du canal d'eau résiduaire sur l'installation de remplissage (5), et réciproquement, et que, au moment où une deuxième valeur de la conductance, inférieure à la première, est atteinte, ces vannes (2, 4) sont commutées du canal d'eau résiduaire sur le réservoir recevant les boissons restante, et réciproquement.

7. Procédé selon la revendication 6, caractérisé par le fait que pour les liquides ayant des conductances semblables, on commute au moment où l'on atteint la valeur identique de la conductance, première et/ou deuxième.

8. Procédé selon la revendication 1, caractérisé par le fait que les signaux de mesure sont analysés par l'intermédiaire d'une commande librement programmable avec traitement des valeurs analogiques.

9. Procédé selon la revendication 1, caractérisé par le fait que la conductance est mesurée simultanément en plusieurs points de mesure de la conduite collective et que les vannes (2, 3, 4) sont commutées en fonction des écarts de conductance à ces points de mesure.
